# EUROPEAN PATENT APPLICATION

(11) **EP 4 821 597 A1**
(43) Date of publication of application: **14.10.2026**
(21) Application number: 24925645.4
(22) Date of filing: 25.12.2024
(51) Int. Cl.: B01J 29/08, C07D 313/04

(54) **SN-Y TYPE MOLECULAR SIEVE, PREPARATION METHOD THEREFOR AND USE THEREOF, AND OXIDATION REACTION METHOD FOR CYCLIC KETONE**

(30) Priority: 20.02.2024 CN 202410187144
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: XUE, Jinghang, Dalian, Liaoning 116045 (CN); QIN, Bo, Dalian, Liaoning 116045 (CN); LIU, Wei, Dalian, Liaoning 116045 (CN); ZHANG, Tong, Dalian, Liaoning 116045 (CN); ZHANG, Xiaoping, Dalian, Liaoning 116045 (CN)
(74) Representative: Hoffmann Eitle
(86) International application number: PCT/CN2024/142257
(87) International publication number: WO 2025/175908

(57) **Abstract**

An Sn-Y type molecular sieve, a preparation method therefor and the use thereof, and an oxidation reaction method for a cyclic ketone. The surface silicon/tin molar ratio of the molecular sieve is 50-70, and the bulk silicon/tin molar ratio is 200-300; with respect to the Sn-Y type molecular sieve, Sn is mainly intensively distributed on the outer surface of a Y molecular sieve, thus achieving better catalytic performance for the Baeyer-Villiger oxidation reaction of a cyclic ketone.

## Description

### CROSS-REFERENCE TO RELATED APPLICATIONS

The application claims the benefit of China patent application No. "202410187144.6", filed on February 20, 2024, the content of which is specifically and entirely incorporated herein by reference.

### TECHNICAL FIELD

The present invention relates to the field of molecular sieve synthesis, in particular to an Sn-Y type molecular sieve, a preparation method therefor and the use thereof, and an oxidation reaction method for a cyclic ketone.

### BACKGROUND ART

Heteroatom molecular sieves refer to the molecular sieves containing other elements formed by isomorphously replacing a portion of silicon, aluminum, or phosphorus atoms in the molecular sieve skeleton with other heteroatoms. Because the introduction of heteroatoms into the molecular sieve skeleton often causes a certain degree of change in the original structure and properties of the molecular sieve (e.g., acidity, particle size, pore properties), it has gradually overcome the limitations on the use of traditional silicon-aluminum molecular sieves and broadened the application fields of molecular sieves in recent years. The synthesis of heteroatom molecular sieves is also regarded as an important branch of the molecular sieve synthesis field with potential commercial value, and has attracted increasing attention.

Since the successful synthesis of TS-1 molecular sieve, researchers have prepared a variety of molecular sieves containing heteroatoms (e.g., tin, iron, gallium, zirconium and vanadium). Among them, the synthesis of tin-based molecular sieves has attracted considerable attention. Because Sn is embedded in the molecular sieve skeleton, it provides the molecular sieve with unique Lewis acid centers, which can catalyze the Baeyer-Viiliger reaction of ketones. Sn-beta molecular sieve was synthesized in 1997, and the Sn-beta molecular sieve was successfully used for catalyzing the Baeyer-Viiliger reaction of cyclohexanone in 2001, the catalyst exhibited desirable selectivity for the product ε-caprolactone. However, given that the Baeyer-Viiliger reaction of cyclohexanone typically uses a certain concentration of hydrogen peroxide as an oxidant, the reaction inevitably introduces water, which will be adsorbed on the active Sn centers, resulting in the problems such as the degraded catalytic performance and increased byproducts. Therefore, the use of a solvent containing tert-butyl hydroperoxide for replacing the hydrogen peroxide as an oxidant has become a new research direction in recent years. However, due to the large particle size of tert-butyl hydroperoxide molecules, the diffusion restriction effect of Sn-beta molecular sieve pores is more pronounced, the practical application effects of the molecular sieve is not satisfactory. Y zeolites have more open pores and a supercage structure, the synthesis of Sn-Y molecular sieve has been reported in recent years.

Zhiguo Zhu et al. reported a method for synthesizing Sn-Y molecular sieves in the literature "Postsynthesis and Effective Baeyer-Villiger Oxidation Properties of Hierarchical FAU-type Stannosilicates", the Journal of Physical Chemistry C, Vol. 120. The method comprises the following steps: first preparing a USY molecular sieve, then placing USY powder in an aqueous solution of (NH₄)₂SnCl₆, and adding hydrochloric acid to control pH of the reaction, carrying out the reaction under a stirring condition at a constant temperature for a period of time to produce the Sn-Y molecular sieve. However, the tin in the molecular sieve skeleton synthesized with the method is primarily distributed within the molecular sieve, which is not conducive to contacting of the active sites of the Sn species with the reactant molecules during the reaction process.

Zhiguo Zhu et al. reported a method for synthesizing Sn-Y molecular sieves in the literature "Postsynthesis of FAU-type stannosilicate as efficient heterogeneous catalyst for Baeyer-Villiger oxidation", the APPLIED CATALYSIS A-GENERAL, Vol. 519. The method uses a USY molecular sieve as the raw material, the USY molecular sieve is first subjected to an acid treatment, and Sn is then implanted into the USY molecular sieve by reacting the dealuminated USY molecular sieve with SnCl₄ at a high temperature. However, the Sn atoms in the Sn-Y molecular sieve synthesized with the method are mainly distributed inside the molecular sieve, and the Sn atoms are not sufficiently exposed, which is not conducive to contacting of the active sites of the Sn species with the reactant molecules during the reaction process.

### SUMMARY OF THE INVENTION

In order to overcome the defects in the prior art, the present invention provides an Sn-Y type molecular sieve, a preparation method therefor and the use thereof, and an oxidation reaction method for a cyclic ketone. The Sn in the Sn-Y molecular sieve provided by the present invention is mainly intensively distributed on an outer surface of the Y molecular sieve, thereby achieving the improved activity and selectivity for the Baeyer-Villiger oxidation reaction of a cyclic ketone.

The first aspect of the present invention provides an Sn-Y type molecular sieve, wherein the molecular sieve has a surface silicon/tin molar ratio within the range of 50-70 and a bulk phase silicon/tin molar ratio within the range of 200-300.

Preferably, the pyridine infrared L acid content of the molecular sieve at a desorption temperature of 200°C is within the range of 100-200 µmol/g, more preferably within the range of 130-170 µmol/g.

In the Sn-Y molecular sieve provided by the present invention, the skeleton Sn species are mainly concentrated on the outer surface of the Y molecular sieve, and the internal Sn species are relatively small, so that the Lewis acidic sites of the Sn-Y molecular sieve of the present invention are mainly concentrated on the surface of said molecular sieve, which has better accessibility to the reactant molecules and is almost not limited by the diffusion rate during the reaction process, the Sn species providing the Lewis acidic sites are efficiently utilized.

The second aspect of the present invention provided a method for preparing an Sn-Y type molecular sieve, the method comprises the following steps:
1) Subjecting a NaY molecular sieve and an ammonium salt to a solid phase ion exchange, then performing calcination to obtain a HY molecular sieve;
2) Subjecting the HY molecular sieve and a tin source to a solid phase ion exchange to obtain a Y molecular sieve following an ion exchange of Sn;
3) Treating the Y molecular sieve following an ion exchange of Sn in a mixed gas containing water vapor and oxygen gas, and then performing an acid treatment.

The method provided by the present invention first uses ammonium salt and Na⁺ to perform a solid phase ion exchange, and exchange Na⁺ on an outer surface of the Y molecular sieve with quaternary ammonium cations. The quaternary ammonium cations are converted into H⁺ during the subsequent calcination process, and Sn²⁺ is then exchanged onto an outer surface of the Y molecular sieve by means of the solid state ion exchange. Subsequently, through the high-temperature treatment with a mixture of water vapor and oxygen gas, vacancies are formed by dealumination on the outer surface, allowing Sn ions to be inserted into the vacancies on the outer surface of the Y molecular sieve to form a four-coordinated Sn skeleton, ultimately achieving the purpose of intensively distributing Sn species with L acid on the outer surface of said molecular sieve.

The third aspect of the present invention provides a use of the Sn-Y type molecular sieve according to the first aspect in an oxidation reaction of cyclic ketone.

The fourth aspect of the present invention provides an oxidation reaction method for a cyclic ketone, the method comprises: reacting a cyclic ketone with an oxidant in the presence of a solvent and a catalyst under oxidation reaction conditions; wherein the catalyst is the Sn-Y molecular sieve according to the first aspect.

The Sn-Y molecular sieve provided by the present invention has better activity in the oxidation reaction of cyclic ketones and high selectivity and yield for the reaction product lactone. In addition, the Sn-Y molecular sieve prepared with the method of the present invention has a simple operation and a low preparation cost, and can meet the needs of industrial production.

### DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point values of the various ranges, and the individual point values may be combined with one another to produce one or more new numerical ranges, which should be deemed to have been specifically disclosed herein.

Unless otherwise specified in the present invention, all the percentages and contents are based on mass.

The first aspect of the present invention provides an Sn-Y type molecular sieve, wherein the molecular sieve has a surface silicon/tin molar ratio within the range of 50-70 and a bulk phase silicon/tin molar ratio within the range of 200-300. For example, the molecular sieve has an surface silicon/tin molar ratio of 50, 51, 52, 53, 54, 55, 56, 57, 58, 59, 60, 61, 62, 63, 64, 65, 66, 67, 68, 69, 70, more preferably within the range of 55-65. For example, the molecular sieve has a bulk phase silicon/tin molar ratio of 200, 210, 220, 230, 240, 250, 260, 270, 280, 290, 300, more preferably within the range of 230-280.

The Sn-Y type molecular sieve provided by the present invention has a smaller surface silicon/tin molar ratio, and the tin species are mainly distributed on the outer surface of the molecular sieve, which is more conducive to improving the utilization rate of Sn. Moreover, when the Sn-Y type molecular sieve is used in an oxidation reaction of cyclic ketone, it has higher catalytic performance.

In the invention, the term "Sn-Y type molecular sieve" does not specifically limit the properties and structure of the product provided by the invention, as long as the Y type molecular sieve contains Sn element, and the surface silicon-tin molar ratio and the bulk phase silicon-tin molar ratio meet the requirements of the invention and fall into the protection scope of the invention.

The crystal form of the molecular sieve can be determined by the X-Ray Diffraction (XRD) characterization.

According to the present invention, preferably, the molecular sieve has a bulk phase SiO₂/Al₂O₃ molar ratio within the range of 60-140, more preferably within the range of 80-120, such as 80, 90, 100, 110 or 120.

According to the present invention, preferably, the molecular sieve has a surface SiO₂/Al₂O₃ molar ratio within the range of 350-700, more preferably within the range of 420-630, such as 420, 450, 500, 530, 550, 580, 600 or 630.

In the invention, the surface silicon/tin molar ratio and the surface silica/alumina molar ratio of the molecular sieve are characterized by X-ray Photoelectron Spectroscopy (XPS). Specifically, the XPS characterization is performed on a Kratos Axis ultra X-ray photoelectron spectrometer, with an Al Kα (1486.6 eV) excitation source. The binding energy in the XPS spectrogram is corrected with C 1s (284.8 eV), the surface silicon/tin molar ratio and the surface silica/alumina molar ratio of the molecular sieve are then obtained by conversion.

In the invention, the bulk phase silicon/tin molar ratio of the molecular sieve and the SiO₂/Al₂O₃ molar ratio of the molecular sieve are measured by Inductively Coupled Plasma Atomic Emission Spectroscopy (ICP-AES). Before the test, the molecular sieve solid powder is pretreated and added into a mixed solution of water, nitric acid and hydrofluoric acid, the powder is heated at 150°C until the sample is completely dissolved and becomes a transparent solution. After cooling, it is transferred to a volumetric flask, deionized water is added to a fixed volume and shaken. Subsequently, quantitative analysis is performed using a standard curve method. Finally, the measured corresponding element content is converted into the forms of silica/alumina molar ratio (SiO₂/Al₂O₃) and silicon/tin molar ratio (SiO₂/SnO₂).

According to the present invention, preferably, the pyridine infrared L acid content of the molecular sieve at a desorption temperature of 200°C is within the range of 100-200 µmol/g, for example, 100 µmol/g, 110 µmol/g, 120 µmol/g, 130 µmol/g, 140 µmol/g, 150 µmol/g, 160 µmol/g, 170 µmol/g, 180 µmol/g, 190 µmol/g, 200 µmol/g, more preferably within the range of 130-170 µmol/g. The molecular sieve provided by the present invention has a higher pyridine infrared L acid content at a desorption temperature of 200°C.

According to the present invention, preferably, the pyridine infrared L acid/B acid content ratio of the molecular sieve at a desorption temperature of 200°C is within the range of 90-130, such as 90, 100, 110, 120, 130, and more preferably within the range of 110-120. An use of the molecular sieve with an appropriate L acid/B acid content ratio is more conducive to improving the conversion rate and target product selectivity during the reaction process.

In the present invention, the contents of L acid and B acid in the molecular sieve are measured by pyridine-infrared spectroscopy. Specifically, the sample is prepared into a self-supporting sheet, heated to 500°C and maintained the temperature for 2 hours, and then vacuumed. The sample is naturally cooled to room temperature and then adsorbed with pyridine at room temperature for 30 minutes. The sample is then heated to 200°C and vacuumed to remove the adsorbed pyridine molecules, the infrared spectrum of pyridine at the temperature is recorded. The peak at 1450 cm⁻¹ in the pyridine infrared spectrum is attributed to the L acid absorption peak, and the peak at 1545 cm⁻¹ is attributed to the B acid absorption peak. After the peak area of the spectrogram is subjected to an integration treatment, the infrared L acid and B acid contents are obtained.

The quantitative calculation of L acid can be obtained with the following formula:
L Site density $\left(μmol/g\right) = \frac{{I}_{A \left({cm}^{- 1}\right)}}{E \left(cm/μmol\right)} \times \frac{a \left({cm}^{2}\right)}{m \left(g\right)}$; wherein *I_{A}* denotes the integrated area of the absorption peak at 1450 cm⁻¹ in the spectrogram; a and m denote the area and mass of the sample self-supporting sheet respectively; E denotes the integrated molar extinction coefficient, E = 1.42 cm/µmol.

The quantitative calculation of B acid can be obtained with the following formula:
B Site density $\left(μmol/g\right) = \frac{{I}_{A \left({cm}^{- 1}\right)}}{E \left(cm/μmol\right)} \times \frac{a \left({cm}^{2}\right)}{m \left(g\right)}$; wherein *I_{A}* denotes the integrated area of the absorption peak at 1545 cm⁻¹ in the spectrogram; a and m denote the area and mass of the sample self-supporting sheet respectively; E denotes the integrated molar extinction coefficient, E = 1.95 cm/µmol.

According to the present invention, preferably, the molecular sieve has a specific surface area within the range of 770-850 m²/g, more preferably within the range of 790-830 m²/g.

According to the present invention, preferably, the molecular sieve has a pore volume within the range of 0.36-0.43 mL/g, more preferably within the range of 0.37-0.41 mL/g.

The molecular sieve provided by the invention has a large specific surface area and a large pore volume. In the invention, the specific surface area and pore volume of the molecular sieve are measured using the N₂-adsorption and desorption method. Before measurement, the sample is first pretreated at 300°C for 3 hours and then tested by nitrogen adsorption at 77K. The specific surface area of the molecular sieve is calculated using the BET method, and the total pore volume is measured at p/p⁰ = 0.98.

According to the present invention, preferably, the molecular sieve has a relative crystallinity within the range of 86-96%, more preferably within the range of 89-93%. The molecular sieve provided by the present invention has a higher crystallinity, which is more conducive to improving its performance in the catalytic oxidation reaction of cyclic ketones.

In the invention, the relative crystallinity of the Sn-Y molecular sieve sample is measured by using the X-ray diffraction, specifically measured with the method in the China Petrochemical Industry Standard SH/T 0340-92.

The molecular sieves having the aforementioned properties of the invention can achieve the purpose of the invention, and there are no special requirements for the preparation methods of said molecular sieve. According to a preferred embodiment of the present invention, the second aspect of the present invention provides a method for preparing an Sn-Y type molecular sieve, the method comprises the following steps:
1) Subjecting a NaY molecular sieve and an ammonium salt to a solid phase ion exchange, then performing calcination to obtain a HY molecular sieve;
2) Subjecting the HY molecular sieve and a tin source to a solid phase ion exchange to obtain a Y molecular sieve following an ion exchange of Sn;
3) Treating the Y molecular sieve following an ion exchange of Sn in a mixed gas containing water vapor and oxygen gas, and then performing an acid treatment.

In the present invention, the NaY molecular sieve raw material can be a commercially available product or any NaY molecular sieve prepared according to the prior art. Preferably, the mass content of Na₂O in the NaY molecular sieve in step 1) is within the range of 10.5-12%, more preferably within the range of 10.8-11.7%. Preferably, the NaY molecular sieve has a SiO₂/Al₂O₃ molar ratio within the range of 4.8-5.7, more preferably within the range of 5.0-5.5.

According to the method provided by the invention, the Na₂O content in the molecular sieve is measured by X-ray fluorescence (XRF).

The present invention has a wide selection range for the ammonium salt, as long as it can carry out ammonium exchange with the NaY molecular sieve, the ammonium salt may be an inorganic ammonium, or an organic ammonium, or a mixture thereof, preferably organic ammonium. In order to further improve the catalytic performance of the molecular sieve, preferably, the ammonium salt is at least one selected from the group consisting of organic quaternary ammonium salts. The anions of the organic quaternary ammonium salts may be halide ions, include but not limited to bromide ion and/or chloride ion. The invention has as wide selection range for the group connected with N, for example, it may be C1-C5 alkyl, and the four alkyls that are connected with N may be identical or different.

According to a preferred embodiment of the present invention, the ammonium salts are at least one selected from the group consisting of tetramethylammonium bromide, tetramethylammonium chloride, tetraethylammonium bromide, tetraethylammonium chloride, tetrapropylammonium bromide, tetrapropylammonium chloride, propyltrimethylammonium bromide and propyltrimethylammonium chloride, more preferably tetramethylammonium bromide and/or tetramethylammonium chloride. The preferred embodiment is more conducive to preferentially exchanging sodium ions on the outer surface of the molecular sieve.

In the present invention, the solid phase ion exchange is used for exchanging the Na ions of the NaY molecular sieve with the ammonium of the ammonium salt. The solid phase ion exchange has a conventional meaning in the field, specifically referring to that the ion exchange is carried out under solid phase conditions, and there is no solvent (e.g., water) during the exchange process. The inventors of the present invention have found in the course of research that by carrying out solid phase ion exchange of the NaY molecular sieve with the ammonium salt, the Na⁺ on the outer surface of the Y molecular sieve can be preferentially exchanged for quaternary ammonium cations by controlling the solid phase ion exchange conditions, and combined with the other steps of the present invention, it is more conducive to achieving the purpose of centrally distributing Sn species with L acid on an outer surface of the molecular sieve.

According to the present invention, preferably, the dosage ratio by mass of said ammonium salt to said NaY molecular sieve is within the range of 0.04-0.14, more preferably within the range of 0.07-0.1.

According to a preferred embodiment of the present invention, in order to mix the ammonium salt and the NaY molecular sieve more uniformly, preferably, the ammonium salt and the NaY molecular sieve are ground under an inert atmosphere (e.g., a glove box) before the solid phase ion exchange.

According to the present invention, preferably, the conditions for said solid phase ion exchange in step 1) comprise: a reaction temperature within the range of 140-190°C, more preferably within the range of 160-170°C, and a reaction time of 60-180 min, more preferably 100-140 min under an inert atmosphere. The inert atmosphere is provided by an inert gas commonly used in the field, preferably one selected from nitrogen, helium, argon, or a mixture thereof in any proportion.

According to a specific embodiment of the invention, the solid phase ion exchange is carried out under an inert gas circulation state.

According to a preferred embodiment of the present invention, step 1) of the method comprises: placing a NaY molecular sieve and ammonium salt in a reactor, introducing an inert gas into one end of the reactor and emptying the other end, maintaining the inert gas circulation state in the reactor, and carrying out the reaction at the temperature of solid phase ion exchange.

According to the invention, there is no particular limitation on the specific mode of performing the solid phase ion exchange in step 1), the solid phase ion exchange can be performed in a tubular furnace reactor.

Preferably, the method further comprises: washing, filtering and drying the solid product obtained by the solid-phase ion exchange, before the calcination in step 1). The invention does not particularly define the conditions for washing, filtering and drying, those skilled in the art can select the conditions according to conventional technical means.

According to the present invention, the calcination conditions in step 1) can be selected from a wide range, as long as the quaternary ammonium cation can be converted to H⁺ during the calcination process. Preferably, the calcination conditions in step 1) comprise: a calcination temperature within the range of 500-550°C and a calcination time of 3-6 hours in an oxygen-containing atmosphere. The present invention does not impose particular limitation to the oxygen-containing atmosphere, it may be air, for example.

According to the present invention, preferably, the method further comprises drying and/or calcining the NaY molecular sieve and the ammonium salt independently before performing the solid phase ion exchange. Drying and/or calcining the NaY molecular sieve and the ammonium salt to remove the water carried by the NaY molecular sieve and the ammonium salt, it is more conducive to ensuring that step 1) is carried out under anhydrous conditions. In a case of preferably, the ammonium salt is dried and the NaY molecular sieve is calcined. More preferably, the calcination conditions for the NaY molecular sieve comprise: a calcination temperature within the range of 500-550°C and a calcination time of 3-6h. More preferably, the drying conditions for the ammonium salt comprise: a drying temperature within the range of 100-120°C and a drying time of 4-7h.

According to the present invention, in step 2), in order to mix the HY molecular sieve and the tin source more uniformly, preferably, the HY molecular sieve and the tin source are ground under an inert atmosphere (e.g., a glove box) before the solid phase ion exchange in step 2).

The specific operation mode of the solid phase ion exchange and the selection range of the reactor in step 2) may be the same as those in step 1), the content will not be repeatedly described herein.

According to the present invention, preferably, the mass ratio of said tin source and said HY molecular sieve in step 2) is within the range of 0.07-0.15, more preferably within the range of 0.1-0.12. An use of the preferred embodiment is more conducive to exchanging Sn²⁺ onto an outer surface of the Y molecular sieve.

According to the present invention, preferably, the conditions for said solid phase ion exchange in step 2) comprise: a reaction temperature within the range of 250-350°C, more preferably within the range of 270-330°C, and a reaction time of 60-180 min, more preferably 100-140 min under an inert atmosphere. The inert atmosphere can be selected from the range as described above.

According to the present invention, preferably, the tin source is an anhydrous tin source, more preferably a divalent tin source, further preferably at least one selected from the group consisting of stannous chloride, stannous sulfate and tin (II) acetate.

Preferably, the method further comprises: washing, filtering, and drying the solid product obtained by solid-phase ion exchange in step 2) before step 3). The invention does not particularly limit the conditions for washing, filtering, and drying, those skilled in the art can select the conditions according to conventional technical means.

According to the present invention, preferably, the molar ratio of water vapor to oxygen gas in the mixed gas in step 3) is within the range of 7-9, more preferably within the range of 7.5-8.5. The preferred embodiment is more conducive to embedding tin into the Y molecular sieve skeleton.

According to the present invention, preferably, the conditions for treatment in a mixed gas containing water vapor and oxygen gas in step 3) comprise: a reaction temperature within the range of 550-650°C, more preferably within the range of 580-620°C; a treatment time of 1-4 hours, preferably 2-3 hours; a total pressure within the range of 0.05-0.15MPa, more preferably within the range of 0.08-0.12MPa.

According to a preferred embodiment of the invention, the step of treating the Y molecular sieve following an ion exchange of Sn in a mixed gas containing water vapor and oxygen gas comprises: introducing a mixed gas of water vapor and oxygen gas into a reactor (e.g., a tubular furnace reactor), and then treating the Y molecular sieve following an ion exchange of Sn under a treatment temperature and pressure; after the treatment is completed, the introduction of gas is stopped, take it out after cooling, and subjected to an acid treatment.

The specific conditions and operation methods of the acid treatment in step 3) are not particularly limited in the present invention, the acid treatment in step 3) can remove aluminum from the molecular sieve. The acid used in the acid treatment of step 3) can be selected from a wide range of acids, including inorganic acids and/or organic acids, preferably inorganic acids. The inorganic acid is preferably at least one of nitric acid, hydrochloric acid and sulfuric acid. The organic acid is preferably at least one of citric acid, oxalic acid, Ethylene Diamine Tetraacetic Acid (EDTA) and tartaric acid.

According to the present invention, preferably, the acid concentration used in said acid treatment is within the range of 0.2mol/L-0.4mol/L, more preferably within the range of 0.25-0.35mol/L.

According to the present invention, preferably, the conditions of said acid treatment comprise: a treatment temperature within the range of 30-60°C, preferably within the range of 40-50°C; a treatment time of 60-180 min, more preferably 100-140 min; a solid-liquid mass ratio during said acid treatment is within the range of 1:(6-12), more preferably 1:(8-10). The present invention does not particularly limit the number of acid treatments, it can be one or more times, preferably 1-2 times.

Preferably, the method further comprises: after the acid treatment in step 3), washing, filtering, and drying the solid product obtained from the acid treatment. The invention does not particularly limit the conditions for washing, filtering, and drying, those skilled in the art can select the conditions according to conventional technical means. The drying conditions preferably comprise a drying temperature within the range of 120-160°C and a time of 4-6 hours.

According to the present invention, preferably, the conditions for solid phase ion exchange of a NaY molecular sieve with an ammonium salt and the conditions for solid phase ion exchange of the HY molecular sieve with a tin source cause that the prepared molecular sieve has a surface silicon/tin molar ratio within the range of 50-70 and a bulk phase silicon/tin molar ratio within the range of 200-300.

The third aspect of the present invention provides an use of the Sn-Y molecular sieve according to the first aspect mentioned above in an oxidation reaction of cyclic ketone. The Sn-Y molecular sieve provided by the present invention has high performance in the oxidation reaction of cyclic ketone, in particular the Baeyer-Villiger oxidation reaction. Preferably, the Sn-Y molecular sieve is used in the Baeyer-Villiger oxidation reaction of cyclic ketones. The Sn-Y molecular sieve of the present invention is suitable for the oxidation reaction of a variety of cyclic ketones, such as at least one of cyclohexanone, 2-adamantanone, dihydrocarvone and bicyclo[3,2,0]hept-2-en-6-one, and preferably the Sn-Y molecular sieve is used in the oxidation reaction of cyclohexanone or 2-adamantanone.

The fourth aspect of the present invention provides an oxidation reaction method for a cyclic ketone, the method comprises: reacting a cyclic ketone with an oxidant in the presence of a solvent and a catalyst under oxidation reaction conditions; wherein the catalyst is the Sn-Y molecular sieve according to the first aspect.

Preferably, the cyclic ketone is at least one of cyclohexanone, 2-adamantanone, dihydrocarvone and bicyclo[3,2,0]hept-2-en-6-one, more preferably cyclohexanone and/or 2-adamantanone.

The present invention has a wide selection range for the oxidant, the oxidant may be any conventional choice in the field, as long as it can oxidize the cyclic ketone. Preferably, the oxidant is at least one selected from the group consisting of tert-butyl hydroperoxide, hydrogen peroxide, cumene hydroperoxide, cyclohexyl hydroperoxide, peracetic acid and peroxypropionic acid, more preferably tert-butyl hydroperoxide.

The present invention has a wide selection range for the type of solvent, as long as it can provide a reaction environment. Preferably, the solvent is at least one selected from the group consisting of 1,4-dioxane, decane, chlorobenzene and benzyl alcohol.

Preferably, the molar ratio of said oxidant to said cyclic ketone is 1:(1-3).

Preferably, the catalyst is used in an amount of 80-600mg, more preferably 80-300mg, relative to 1 mmol of said oxidant.

Preferably, the oxidation reaction conditions comprise: an oxidation reaction temperature within the range of 75-95°C, and an oxidation reaction time of 2-8 hours.

It should be noted that unless otherwise specified in the present invention, all percentages and contents are based on mass.

The preparation process and product performance of the molecular sieve of the present invention are further described below with reference to examples and comparative examples, but the following examples do not impose limitations to the method of the present invention.

In the following examples and comparative examples, the surface silicon/tin molar ratio of the molecular sieves was measured by X-ray Photoelectron Spectroscopy (XPS), and the bulk phase silicon/tin molar ratio and the SiO₂/Al₂O₃ molar ratio of the molecular sieve were tested by the Inductively Coupled Plasma Atomic Emission Spectroscopy (ICP-AES). The L acid and B acid contents of the molecular sieve were measured by the pyridine-infrared spectroscopy. The specific surface area and pore volume of the molecular sieve were measured by N₂-adsorption and desorption method. The relative crystallinity of the molecular sieve was measured with the method specified by the China Petrochemical Industry Standard SH/T 0340-92. The Na₂O content in the molecular sieve was measured by the X-Ray Fluorescence (XRF). The specific conditions of each test method were as described in the DESCRIPTION OF THE PREFERRED EMBODIMENT mentioned above and were not be repeatedly described herein.

### Example 1

A NaY molecular sieve (with a SiO₂/Al₂O₃ molar ratio of 4.8) containing 12.0% of Na₂O was calcined at a temperature of 500°C for 3 hours, and tetramethylammonium bromide powder was dried at a temperature of 110°C for 5 hours. The NaY molecular sieve and tetramethylammonium bromide powder were then ground and mixed uniformly in a glove box, the mass ratio of tetramethylammonium bromide to NaY molecular sieve was controlled at 0.042; the solid mixture was then loaded into a tubular furnace reactor, helium gas was introduced into the tubular furnace, the circulation condition of inert gas in the reactor was maintained, after an air in the reactor was replaced with helium gas, the internal temperature of said reactor was raised to 185°C and the reaction was carried out for 70 minutes; after the reaction was completed, the reaction product was cooled in a helium atmosphere, then taken out, washed, filtered and dried, a Y molecular sieve following an exchange of quaternary ammonium cations was obtained.

The Y molecular sieve following an exchange of quaternary ammonium cations was calcined in air at a temperature of 530°C for 4 hours, a HY molecular sieve was obtained. Subsequently, the HY molecular sieve and anhydrous stannous chloride powder were ground and mixed uniformly in a glove box, the mass ratio of anhydrous stannous chloride to HY molecular sieve was controlled at 0.07, the solid mixture was then transferred to a tubular furnace reactor; helium gas was introduced into the tubular furnace, the circulation condition of inert gas in the reactor was maintained, after an air in the reactor was replaced with helium gas, the internal temperature of said reactor was raised to 345°C and the reaction was carried out for 60 minutes; after the reaction was completed, the reaction product was cooled in a helium atmosphere, taken out, washed, filtered and dried, a Y molecular sieve Sn(II)Y following an ion exchange of Sn was obtained.

The Sn(II)Y molecular sieve was loaded into a tubular furnace reactor, a mixed gas consisting of water vapor and oxygen gas with a molar ratio of 7.0 was introduced, the reaction was carried out at a temperature of 650°C for 1 hour, and the total pressure in the reactor was controlled at 0.06 MPa; after the treatment was completed, the introduction of gas was stopped, the reaction product was cooled and then taken out, a Sn-Y molecular sieve was obtained.

The obtained Sn-Y molecular sieve was placed in a hydrochloric acid solution with a concentration of 0.2 mol/L and a temperature of 60°C and treated for 170 minute, the solid-liquid mass ratio was controlled at 1:12 during the treatment, and the treatment was performed once; the treated solid was filtered, washed, and dried at 130°C for 4 hours, the final Sn-Y molecular sieve product was prepared.

### Example 2

A NaY molecular sieve (with a SiO₂/Al₂O₃ molar ratio of 5.7) containing 10.6% of Na₂O was calcined at a temperature of 520°C for 4 hours, and tetramethylammonium bromide powder was dried at a temperature of 110°C for 5 hours. The NaY molecular sieve and tetramethylammonium bromide powder were then ground and mixed uniformly in a glove box, the mass ratio of tetramethylammonium bromide to NaY molecular sieve was controlled at 0.13; the solid mixture was then loaded into a tubular furnace reactor, helium gas was introduced into the tubular furnace, the circulation condition of inert gas in the reactor was maintained, after an air in the reactor was replaced with helium gas, the internal temperature of said reactor was raised to 144°C and the reaction was carried out for 170 minutes; after the reaction was completed, the reaction product was cooled in a helium atmosphere, then taken out, washed, filtered and dried, a Y molecular sieve following an exchange of quaternary ammonium cations was obtained.

The Y molecular sieve following an exchange of quaternary ammonium cations was calcined in air at a temperature of 520°C for 5 hours, a HY molecular sieve was obtained. Subsequently, the HY molecular sieve and anhydrous stannous chloride powder were ground and mixed uniformly in a glove box, the mass ratio of anhydrous stannous chloride to HY molecular sieve was controlled at 0.14, the solid mixture was then transferred to a tubular furnace reactor; helium gas was introduced into the tubular furnace, the circulation condition of inert gas in the reactor was maintained, after an air in the reactor was replaced with helium gas, the internal temperature of said reactor was raised to 260°C and the reaction was carried out for 177 minutes; after the reaction was completed, the reaction product was cooled in a helium atmosphere, taken out, washed, filtered and dried, a Y molecular sieve Sn(II)Y following an ion exchange of Sn was obtained.

The Sn(II)Y molecular sieve was loaded into a tubular furnace reactor, a mixed gas consisting of water vapor and oxygen gas with a molar ratio of 8.8 was introduced, the reaction was carried out at a temperature of 560°C for 3.5 hours, and the total pressure in the reactor was controlled at 0.14 MPa; after the treatment was completed, the introduction of gas was stopped, the reaction product was cooled and then taken out, a Sn-Y molecular sieve was obtained.

The obtained Sn-Y molecular sieve was placed in a hydrochloric acid solution with a concentration of 0.4 mol/L and a temperature of 35°C and treated for 90 minute, the solid-liquid mass ratio was controlled at 1:7 during the treatment, and the treatment was performed twice; the treated solid was filtered, washed, and dried at 150°C for 4 hours, the final Sn-Y molecular sieve product was prepared.

### Example 3

A NaY molecular sieve (with a SiO₂/Al₂O₃ molar ratio of 4.8) containing 12.0% of Na₂O was calcined at a temperature of 550°C for 6 hours, and tetramethylammonium bromide powder was dried at a temperature of 120°C for 5 hours. The NaY molecular sieve and tetramethylammonium bromide powder were then ground and mixed uniformly in a glove box, the mass ratio of tetramethylammonium bromide to NaY molecular sieve was controlled at 0.11; the solid mixture was then loaded into a tubular furnace reactor, helium gas was introduced into the tubular furnace, the circulation condition of inert gas in the reactor was maintained, after an air in the reactor was replaced with helium gas, the internal temperature of said reactor was raised to 155°C and the reaction was carried out for 160 minutes; after the reaction was completed, the reaction product was cooled in a helium atmosphere, then taken out, washed, filtered and dried, a Y molecular sieve following an exchange of quaternary ammonium cations was obtained.

The Y molecular sieve following an exchange of quaternary ammonium cations was calcined in air at a temperature of 550°C for 6 hours, a HY molecular sieve was obtained. Subsequently, the HY molecular sieve and anhydrous stannous chloride powder were ground and mixed uniformly in a glove box, the mass ratio of anhydrous stannous chloride to HY molecular sieve was controlled at 0.09, the solid mixture was then transferred to a tubular furnace reactor; helium gas was introduced into the tubular furnace, the circulation condition of inert gas in the reactor was maintained, after an air in the reactor was replaced with helium gas, the internal temperature of said reactor was raised to 340°C and the reaction was carried out for 80 minutes; after the reaction was completed, the reaction product was cooled in a helium atmosphere, taken out, washed, filtered and dried, a Y molecular sieve Sn(II)Y following an ion exchange of Sn was obtained.

The Sn(II)Y molecular sieve was loaded into a tubular furnace reactor, a mixed gas consisting of water vapor and oxygen gas with a molar ratio of 7.2 was introduced, the reaction was carried out at a temperature of 570°C for 4 hours, and the total pressure in the reactor was controlled at 0.06 MPa; after the treatment was completed, the introduction of gas was stopped, the reaction product was cooled and then taken out, a Sn-Y molecular sieve was obtained.

The obtained Sn-Y molecular sieve was placed in a nitric acid solution with a concentration of 0.22 mol/L and a temperature of 55°C and treated for 150 minute, the solid-liquid mass ratio was controlled at 1:11 during the treatment, and the treatment was performed twice; the treated solid was filtered, washed, and dried at 150°C for 4 hours, the final Sn-Y molecular sieve product was prepared.

### Example 4

A NaY molecular sieve (with a SiO₂/Al₂O₃ molar ratio of 5.3) containing 11.0% of Na₂O was calcined at a temperature of 530°C for 5 hours, and tetramethylammonium bromide powder was dried at a temperature of 110°C for 6 hours. The NaY molecular sieve and tetramethylammonium bromide powder were then ground and mixed uniformly in a glove box, the mass ratio of tetramethylammonium bromide to NaY molecular sieve was controlled at 0.08; the solid mixture was then loaded into a tubular furnace reactor, helium gas was introduced into the tubular furnace, the circulation condition of inert gas in the reactor was maintained, after an air in the reactor was replaced with helium gas, the internal temperature of said reactor was raised to 162°C and the reaction was carried out for 140 minutes; after the reaction was completed, the reaction product was cooled in a helium atmosphere, then taken out, washed, filtered and dried, a Y molecular sieve following an exchange of quaternary ammonium cations was obtained.

The Y molecular sieve following an exchange of quaternary ammonium cations was calcined in air at a temperature of 530°C for 5 hours, a HY molecular sieve was obtained. Subsequently, the HY molecular sieve and anhydrous stannous chloride powder were ground and mixed uniformly in a glove box, the mass ratio of anhydrous stannous chloride to HY molecular sieve was controlled at 0.10, the solid mixture was then transferred to a tubular furnace reactor; helium gas was introduced into the tubular furnace, the circulation condition of inert gas in the reactor was maintained, after an air in the reactor was replaced with helium gas, the internal temperature of said reactor was raised to 330°C and the reaction was carried out for 110 minutes; after the reaction was completed, the reaction product was cooled in a helium atmosphere, taken out, washed, filtered and dried, a Y molecular sieve Sn(II)Y following an ion exchange of Sn was obtained.

The Sn(II)Y molecular sieve was loaded into a tubular furnace reactor, a mixed gas consisting of water vapor and oxygen gas with a molar ratio of 8.3 was introduced, the reaction was carried out at a temperature of 615°C for 3 hours, and the total pressure in the reactor was controlled at 0.09 MPa; after the treatment was completed, the introduction of gas was stopped, the reaction product was cooled and then taken out, a Sn-Y molecular sieve was obtained.

The obtained Sn-Y molecular sieve was placed in a sulfuric acid solution with a concentration of 0.28 mol/L and a temperature of 43°C and treated for 140 minute, the solid-liquid mass ratio was controlled at 1:10 during the treatment, and the treatment was performed once; the treated solid was filtered, washed, and dried at 140°C for 5 hours, the final Sn-Y molecular sieve product was prepared.

### Example 5

A NaY molecular sieve (with a SiO₂/Al₂O₃ molar ratio of 5.2) containing 11.5% of Na₂O was calcined at a temperature of 520°C for 5 hours, and tetramethylammonium bromide powder was dried at a temperature of 110°C for 5 hours. The NaY molecular sieve and tetramethylammonium bromide powder were then ground and mixed uniformly in a glove box, the mass ratio of tetramethylammonium bromide to NaY molecular sieve was controlled at 0.10; the solid mixture was then loaded into a tubular furnace reactor, helium gas was introduced into the tubular furnace, the circulation condition of inert gas in the reactor was maintained, after an air in the reactor was replaced with helium gas, the internal temperature of said reactor was raised to 170°C and the reaction was carried out for 110 minutes; after the reaction was completed, the reaction product was cooled in a helium atmosphere, then taken out, washed, filtered and dried, a Y molecular sieve following an exchange of quaternary ammonium cations was obtained.

The Y molecular sieve following an exchange of quaternary ammonium cations was calcined in air at a temperature of 530°C for 5 hours, a HY molecular sieve was obtained. Subsequently, the HY molecular sieve and anhydrous stannous chloride powder were ground and mixed uniformly in a glove box, the mass ratio of anhydrous stannous chloride to HY molecular sieve was controlled at 0.12, the solid mixture was then transferred to a tubular furnace reactor; helium gas was introduced into the tubular furnace, the circulation condition of inert gas in the reactor was maintained, after an air in the reactor was replaced with helium gas, the internal temperature of said reactor was raised to 290°C and the reaction was carried out for 135 minutes; after the reaction was completed, the reaction product was cooled in a helium atmosphere, taken out, washed, filtered and dried, a Y molecular sieve Sn(II)Y following an ion exchange of Sn was obtained.

The Sn(II)Y molecular sieve was loaded into a tubular furnace reactor, a mixed gas consisting of water vapor and oxygen gas with a molar ratio of 7.7 was introduced, the reaction was carried out at a temperature of 585°C for 2 hours, and the total pressure in the reactor was controlled at 0.12 MPa; after the treatment was completed, the introduction of gas was stopped, the reaction product was cooled and then taken out, a Sn-Y molecular sieve was obtained.

The obtained Sn-Y molecular sieve was placed in a hydrochloric acid solution with a concentration of 0.35 mol/L and a temperature of 50°C and treated for 105 minute, the solid-liquid mass ratio was controlled at 1:8 during the treatment, and the treatment was performed twice; the treated solid was filtered, washed, and dried at 140°C for 5 hours, the final Sn-Y molecular sieve product was prepared.

### Comparative Example 1

A NaY molecular sieve (with a SiO₂/Al₂O₃ molar ratio of 4.8) containing 12.0% of Na₂O was calcined at a temperature of 500°C for 3 hours, and tetramethylammonium bromide powder was dried at a temperature of 110°C for 5 hours. The NaY molecular sieve was then added into distilled water, the mass ratio of NaY molecular sieve to distilled water was controlled at 1:10; tetramethylammonium bromide was subsequently added, the mass ratio of tetramethylammonium bromide to NaY molecular sieve was controlled at 0.042; an ion exchange was performed at a temperature of 90°C for once, the exchange time was 70min, after completion of ion exchange, the product was taken out, washed, filtered and dried, a Y molecular sieve following an exchange of quaternary ammonium cations was obtained.

The Y molecular sieve following an exchange of quaternary ammonium cations was calcined in air at a temperature of 530°C for 4 hours, a HY molecular sieve was obtained. Subsequently, the HY molecular sieve and anhydrous stannous chloride powder were ground and mixed uniformly in a glove box, the mass ratio of anhydrous stannous chloride to HY molecular sieve was controlled at 0.07, the solid mixture was then transferred to a tubular furnace reactor; helium gas was introduced into the tubular furnace, the circulation condition of inert gas in the reactor was maintained, after an air in the reactor was replaced with helium gas, the internal temperature of said reactor was raised to 345°C and the reaction was carried out for 60 minutes; after the reaction was completed, the reaction product was cooled in a helium atmosphere, taken out, washed, filtered and dried, a Y molecular sieve Sn(II)Y following an ion exchange of Sn was obtained.

The Sn(II)Y molecular sieve was loaded into a tubular furnace reactor, a mixed gas consisting of water vapor and oxygen gas with a molar ratio of 7.0 was introduced, the reaction was carried out at a temperature of 650°C for 1 hour, and the total pressure in the reactor was controlled at 0.06 MPa; after the treatment was completed, the introduction of gas was stopped, the reaction product was cooled and then taken out, a Sn-Y molecular sieve was obtained.

The obtained Sn-Y molecular sieve was placed in a hydrochloric acid solution with a concentration of 0.2 mol/L and a temperature of 60°C and treated for 170 minute, the solid-liquid mass ratio was controlled at 1:12 during the treatment, and the treatment was performed once; the treated solid was filtered, washed, and dried at 130°C for 4 hours, the final Sn-Y molecular sieve product was prepared.

### Comparative Example 2

A NaY molecular sieve (with a SiO₂/Al₂O₃ molar ratio of 4.8) containing 12.0% of Na₂O was calcined at a temperature of 500°C for 3 hours, and tetramethylammonium bromide powder was dried at a temperature of 110°C for 5 hours. The NaY molecular sieve and tetramethylammonium bromide powder were then ground and mixed uniformly in a glove box, the mass ratio of tetramethylammonium bromide to NaY molecular sieve was controlled at 0.042; the solid mixture was then loaded into a tubular furnace reactor, helium gas was introduced into the tubular furnace, the circulation condition of inert gas in the reactor was maintained, after an air in the reactor was replaced with helium gas, the internal temperature of said reactor was raised to 185°C and the reaction was carried out for 70 minutes; after the reaction was completed, the reaction product was cooled in a helium atmosphere, then taken out, washed, filtered and dried, a Y molecular sieve following an exchange of quaternary ammonium cations was obtained.

The Y molecular sieve following an exchange of quaternary ammonium cations was calcined in air at a temperature of 530°C for 4 hours, a HY molecular sieve was obtained. Subsequently, the HY molecular sieve was placed in a stannous chloride solution, the mass ratio of anhydrous stannous chloride to HY molecular sieve in the solution was controlled at 0.07, the solid-liquid mass ratio of the molecular sieve to the stannous chloride solution was 1:5, an exchange was performed at a temperature of 90°C for 2 hours. After completion of the ion exchange, the solid was filtered, washed and dried, a Y molecular sieve Sn(II)Y following an ion exchange of Sn was obtained.

The Sn(II)Y molecular sieve was loaded into a tubular furnace reactor, a mixed gas consisting of water vapor and oxygen gas with a molar ratio of 7.0 was introduced, the reaction was carried out at a temperature of 650°C for 1 hour, and the total pressure in the reactor was controlled at 0.06 MPa; after the treatment was completed, the introduction of gas was stopped, the reaction product was cooled and then taken out, a Sn-Y molecular sieve was obtained.

The obtained Sn-Y molecular sieve was placed in a hydrochloric acid solution with a concentration of 0.2 mol/L and a temperature of 60°C and treated for 170 minute, the solid-liquid mass ratio was controlled at 1:12 during the treatment, and the treatment was performed once; the treated solid was filtered, washed, and dried at 130°C for 4 hours, the final Sn-Y molecular sieve product was prepared.

### Comparative Example 3

A NaY molecular sieve (with a SiO₂/Al₂O₃ molar ratio of 4.8) containing 12.0% of Na₂O was calcined at a temperature of 500°C for 3 hours, and tetramethylammonium bromide powder was dried at a temperature of 110°C for 5 hours. The NaY molecular sieve and tetramethylammonium bromide powder were then ground and mixed uniformly in a glove box, the mass ratio of tetramethylammonium bromide to NaY molecular sieve was controlled at 0.042; the solid mixture was then loaded into a tubular furnace reactor, helium gas was introduced into the tubular furnace, the circulation condition of inert gas in the reactor was maintained, after an air in the reactor was replaced with helium gas, the internal temperature of said reactor was raised to 185°C and the reaction was carried out for 70 minutes; after the reaction was completed, the reaction product was cooled in a helium atmosphere, then taken out, washed, filtered and dried, a Y molecular sieve following an exchange of quaternary ammonium cations was obtained.

The Y molecular sieve following an exchange of quaternary ammonium cations was calcined in air at a temperature of 530°C for 4 hours, a HY molecular sieve was obtained. The HY molecular sieve was loaded into a tubular furnace reactor, a water vapor was introduced, the reaction was carried out at a temperature of 650°C for 1 hour, and the total pressure in the reactor was controlled at 0.06 MPa; after the treatment was completed, the introduction of gas was stopped, the reaction product was cooled and then taken out, a H-USY molecular sieve was obtained.

The obtained H-USY molecular sieve was placed in a hydrochloric acid solution with a concentration of 0.2 mol/L and a temperature of 60°C and treated for 170 minute, the solid-liquid mass ratio was controlled at 1:12 during the treatment, and the treatment was performed once; the treated solid was filtered, washed, and dried at 130°C for 4 hours, the dealuminated H-USY molecular sieve was obtained.

The dealuminated H-USY molecular sieve was placed in a (NH₄)₂SnCl₆ solution, the mass ratio of (NH₄)₂SnCl₆ to the H-USY molecular sieve in the solution was controlled at 0.07, the solid-liquid mass ratio was 1:80, a certain amount of hydrochloric acid was added, the concentration of hydrochloric acid in the solution was controlled to be 0.1mol/L, the reaction was carried out at a temperature of 70°C for 24 hours, the reaction product was then filtered, dried, and calcined at a temperature of 550°C for 4 hours, the Sn-Y molecular sieve was prepared.

The structure and properties of the molecular sieves prepared in the above Examples and Comparative Examples were listed in Table 1 below.

**Table 1**

| | Specific surface area m²/g | Pore volume mL/g | Relative crystallinity % | Surface silicon/tin ratio | Bulk phase silicon/tin molar ratio |
|---|---|---|---|---|---|
| Example 1 | 843 | 0.36 | 96 | 68 | 296 |
| Example 2 | 772 | 0.42 | 86 | 51 | 202 |
| Example 3 | 780 | 0.37 | 88 | 66 | 220 |
| Example 4 | 827 | 0.38 | 93 | 63 | 276 |
| Example 5 | 795 | 0.40 | 90 | 57 | 240 |
| Comparative Example 1 | 763 | 0.37 | 87 | 188 | 205 |
| Comparative Example 2 | 730 | 0.32 | 88 | 256 | 280 |
| Comparative Example 3 | 741 | 0.33 | 83 | 250 | 274 |

| | | | | | |
|---|---|---|---|---|---|
| Note: the silica/alumina ratio in Table 1 refers to the SiO₂/Al₂O₃ molar ratio. | | | | | |

**Table 1 (continued)**

| | Surface silica/alumina ratio | Bulk phase silica/alumina ratio | L acid content µmol/g | L acid/B acid content ratio |
|---|---|---|---|---|
| Example 1 | 408 | 70 | 105 | 127 |
| Example 2 | 650 | 130 | 198 | 93 |
| Example 3 | 380 | 62 | 126 | 101 |
| Example 4 | 504 | 102 | 135 | 118 |
| Example 5 | 530 | 110 | 167 | 110 |
| Comparative Example 1 | 240 | 254 | 90 | 73 |
| Comparative Example 2 | 360 | 92 | 30 | 98 |
| Comparative Example 3 | 380 | 82 | 42 | 90 |

| | | | | |
|---|---|---|---|---|
| Note: the silica/alumina ratio in Table 1 (continued) refers to the SiO₂/Al₂O₃ molar ratio. | | | | |

### Test Example 1

The test example served to illustrate the properties of molecular sieves provided by the present invention in the Baeyer-Villiger oxidation reaction of cyclohexanone.

The properties of the Sn-Y molecular sieves in the Examples and Comparative Examples were evaluated by the Baeyer-Villiger oxidation reaction of cyclohexanone and tert-butyl hydroperoxide (TBHP). The reaction was carried out in a round-bottom flask equipped with a condensed water cooling device. 250 mg of a catalyst was weighed and added into the flask, 5 mmol of cyclohexanone, 3 mmol of TBHP (decane solution with a concentration of 3.0 mol/L), and 10 mL of 1,4-dioxane (solvent) were subsequently added. The reaction solution was placed in an oil bath pan with a temperature of 85°C to carry out the reaction for 5 hours. After the reaction was completed, the reaction liquid was transferred to an ice water and cooled to room temperature. 0.4 g of chlorobenzene was added as an internal standard, stirred uniformly, and then centrifuged. The supernatant was extracted for analyzing the conversion rate of cyclohexanone, the selectivity and yield of the target product ε-caprolactone by the gas chromatograph. The evaluation results were shown in Table 2.

**Table 2**

| | Cyclohexanone conversion rate,% | ε-caprolactone selectivity, % | ε-caprolactone yield, % |
|---|---|---|---|
| Example 1 | 43.7 | 97.6 | 42.7 |
| Example 2 | 47.2 | 97.0 | 45.8 |
| Example 3 | 44.0 | 96.2 | 42.3 |
| Example 4 | 51.6 | 98.5 | 50.8 |
| Example 5 | 53.8 | 99.2 | 53.4 |
| Comparative Example 1 | 35.5 | 94.4 | 33.5 |
| Comparative Example 2 | 28.8 | 95.2 | 27.4 |
| Comparative Example 3 | 30.1 | 94.0 | 28.3 |

### Test Example 2

The test example served to illustrate the properties of molecular sieves provided by the present invention in the Baeyer-Villiger oxidation reaction of 2-adamantanone.

The properties of the Sn-Y molecular sieves in the Examples and Comparative Examples were evaluated by the Baeyer-Villiger oxidation reaction of 2-adamantanone and tert-butyl hydroperoxide (TBHP). The reaction was carried out in a round-bottom flask equipped with a condensed water cooling device. 550 mg of a catalyst was weighed and added into the flask, 4 mmol of 2-adamantanone, 3 mmol of TBHP (decane solution with a concentration of 6.0 mol/L), and 10 mL of 1,4-dioxane (solvent) were subsequently added. The reaction solution was placed in an oil bath pan with a temperature of 93°C to carry out the reaction for 3 hours. After the reaction was completed, the reaction liquid was transferred to an ice water and cooled to room temperature. 0.4 g of cyclohexanone was added as an internal standard, stirred uniformly, and then centrifuged. The supernatant was extracted for analyzing the conversion rate of 2-adamantanone, the selectivity and yield of the target product lactone by the gas chromatograph. The evaluation results were illustrated in Table 3.

**Table 3**

| | 2-adamantanone conversion rate, % | Lactone (4-oxahomoadamantan-5-one) | |
|---|---|---|---|
| | | Selectivity, % | Yield, % |
| Example 1 | 87.0 | 98.3 | 85.5 |
| Example 2 | 88.3 | 97.6 | 86.2 |
| Example 3 | 87.4 | 98.0 | 85.7 |
| Example 4 | 91.3 | 99.5 | 90.8 |
| Example 5 | 92.2 | 99.7 | 91.9 |
| Comparative Example 1 | 72.6 | 96.4 | 70.0 |
| Comparative Example 2 | 66.8 | 97.1 | 64.9 |
| Comparative Example 3 | 67.2 | 97.0 | 65.2 |

As indicated by the product properties in Table 1 in combination with the evaluation results in Tables 2 and 3, the Sn active species of the Sn-Y molecular sieves provided by the present invention are primarily distributed on an outer surface of the molecular sieves and have a high L acid content. When the molecular sieves are used for catalyzing the oxidation reaction of cyclohexanone, they are conducive to improving the conversion rate of cyclohexanone and the selectivity of the target product ε-caprolactone, resulting in a high ε-caprolactone yield, and exhibit high activity and selectivity during the oxidation reaction of 2-adamantanone, thereby demonstrating excellent catalytic performance.

## Claims

1. An Sn-Y type molecular sieve, is **characterized in that** the molecular sieve has a surface silicon/tin molar ratio within the range of 50-70 and a bulk phase silicon/tin molar ratio within the range of 200-300.

2. The molecular sieve according to claim 1, wherein the molecular sieve has a surface silicon/tin molar ratio within the range of 55-65 and a bulk phase silicon/tin molar ratio within the range of 230-280;
preferably, the molecular sieve has a bulk phase SiO₂/Al₂O₃ molar ratio within the range of 60-140, more preferably within the range of 80-120;
preferably, the molecular sieve has a surface SiO₂/Al₂O₃ molar ratio within the range of 350-700, more preferably within the range of 420-630.

3. The molecular sieve according to claim 1, wherein the pyridine infrared L acid content of the molecular sieve at a desorption temperature of 200°C is within the range of 100-200 µmol/g, preferably within the range of 130-170 µmol/g.

4. The molecular sieve according to claim 1, wherein the pyridine infrared L acid/B acid content ratio of the molecular sieve at a desorption temperature of 200°C is within the range of 90-130, preferably within the range of 110-120.

5. The molecular sieve according to claim 1, wherein the molecular sieve has a specific surface area within the range of 770-850 m²/g, preferably within the range of 790-830 m²/g;
and/or, the molecular sieve has a pore volume within the range of 0.36-0.43 mL/g, preferably within the range of 0.37-0.41 mL/g.

6. The molecular sieve according to any one of claims 1-5, wherein the molecular sieve has a relative crystallinity within the range of 86-96%, preferably within the range of 89-93%.

7. A method for preparing an Sn-Y type molecular sieve, is **characterized in that** the method comprises the following steps:
1) Subjecting a NaY molecular sieve and an ammonium salt to a solid phase ion exchange, then performing calcination to obtain a HY molecular sieve;
2) Subjecting the HY molecular sieve and a tin source to a solid phase ion exchange to obtain a Y molecular sieve following an ion exchange of Sn;
3) Treating the Y molecular sieve following an ion exchange of Sn in a mixed gas containing water vapor and oxygen gas, and then performing an acid treatment.

8. The method according to claim 7, wherein the mass content of Na₂O in the NaY molecular sieve in step 1) is within the range of 10.5-12%, preferably within the range of 10.8-11.7%;
preferably, the NaY molecular sieve has a SiO₂/Al₂O₃ molar ratio within the range of 4.8-5.7, more preferably within the range of 5-5.5.

9. The method according to claim 7, wherein the ammonium salt is at least one selected from the group consisting of organic quaternary ammonium salts, preferably at least one selected from the group consisting of tetramethylammonium bromide, tetramethylammonium chloride, tetraethylammonium bromide, tetraethylammonium chloride, tetrapropylammonium bromide, tetrapropylammonium chloride, propyltrimethylammonium bromide and propyltrimethylammonium chloride, more preferably tetramethylammonium bromide and/or tetramethylammonium chloride.

10. The method according to any one of claims 7-9, wherein the dosage ratio by mass of said ammonium salt to said NaY molecular sieve is within the range of 0.04-0.14, preferably within the range of 0.07-0.1;
preferably, the conditions for said solid phase ion exchange in step 1) comprise: a reaction temperature within the range of 140-190°C, more preferably within the range of 160-170°C, and a reaction time of 60-180 min, more preferably 100-140 min under an inert atmosphere;
preferably, the calcination conditions in step 1) comprise: a calcination temperature within the range of 500-550°C and a calcination time of 3-6 hours in an oxygen-containing atmosphere;
preferably, the method further comprises drying and/or calcining the NaY molecular sieve and the ammonium salt independently before performing the solid phase ion exchange.

11. The method according to any one of claims 7-10, wherein the mass ratio of said tin source and said HY molecular sieve in step 2) is within the range of 0.07-0.15, preferably within the range of 0.1-0.12;
preferably, the conditions for said solid phase ion exchange in step 2) comprise: a reaction temperature within the range of 250-350°C, more preferably within the range of 270-330°C, and a reaction time of 60-180 min, more preferably 100-140 min under an inert atmosphere;
preferably, the tin source is an anhydrous tin source, more preferably a divalent tin source, further preferably at least one selected from the group consisting of stannous chloride, stannous sulfate and tin (II) acetate.

12. The method according to any one of claims 7-11, wherein the molar ratio of water vapor to oxygen gas in the mixed gas in step 3) is within the range of 7-9, preferably within the range of 7.5-8.5;
preferably, the conditions for treatment in a mixed gas containing water vapor and oxygen gas in step 3) comprise: a reaction temperature within the range of 550-650°C, more preferably within the range of 580-620°C; a treatment time of 1-4 hours, preferably 2-3 hours; a total pressure within the range of 0.05-0.15MPa, more preferably within the range of 0.08-0.12MPa.

13. The method according to any one of claims 7-12, wherein the acid in step 3) is an inorganic acid and/or an organic acid, the inorganic acid is preferably at least one of nitric acid, hydrochloric acid and sulfuric acid, and the organic acid is preferably at least one of citric acid, oxalic acid, EDTA and tartaric acid;
preferably, the acid concentration used in said acid treatment is within the range of 0.2mol/L-0.4mol/L, more preferably within the range of 0.25-0.35mol/L;
preferably, the conditions of said acid treatment comprise: a treatment temperature within the range of 30-60°C, preferably within the range of 40-50°C; a treatment time of 60-180 min, more preferably 100-140 min; a solid-liquid mass ratio during said acid treatment is within the range of 1:(6-12), more preferably 1:(8-10).

14. Use of the Sn-Y type molecular sieve according to any one of claims 1-6 in an oxidation reaction of cyclic ketone, preferably use in an oxidation reaction of at least one of cyclohexanone, 2-adamantanone, dihydrocarvone and bicyclo[3,2,0]hept-2-en-6-one.

15. An oxidation reaction method for a cyclic ketone, is **characterized in that** the method comprises: reacting a cyclic ketone with an oxidant in the presence of a solvent and a catalyst under oxidation reaction conditions; wherein the catalyst is the Sn-Y molecular sieve according to any one of claims 1-6;
preferably, the cyclic ketone is at least one of cyclohexanone, 2-adamantanone, dihydrocarvone and bicyclo[3,2,0]hept-2-en-6-one, more preferably cyclohexanone and/or 2-adamantanone;
preferably, the oxidant is at least one selected from the group consisting of tert-butyl hydroperoxide, hydrogen peroxide, cumene hydroperoxide, cyclohexyl hydroperoxide, peracetic acid and peroxypropionic acid, more preferably tert-butyl hydroperoxide;
preferably, the molar ratio of said oxidant to said cyclic ketone is 1:(1-3); preferably, the catalyst is used in an amount of 80-600mg relative to 1 mmol of said oxidant;
preferably, the oxidation reaction conditions comprise: an oxidation reaction temperature within the range of 75-95°C, and an oxidation reaction time of 2-8 hours.
